# DEMANDE DE BREVET EUROPEEN

(11) **EP 1 593 319 A1**
(43) Date de publication de la demande: **09.11.2005**
(21) Numéro de dépôt: 05290898.5
(22) Date de dépôt: 22.04.2005
(51) Int. Cl.: A45D 40/00

(54) **Procédé de maquillage et d'application d'un produit de soin, et dispositifs utilisés dans la mise en oeuvre de tels procédés**

(30) Priorité: 06.05.2004 FR 0404915
(71) Demandeur: L'OREAL, 75008 Paris (FR)
(72) Inventeur: Gueret, Jean-Louis H., 75016 Paris (FR)
(74) Mandataire: Schmit, Charlotte

(57) **Abrégé**

La présente invention concerne un procédé pour l'application, notamment sur des fibres kératiniques, d'un produit cosmétique (P), y compris de soin, comportant des particules (F) de forme allongée telles que des fibres, ce produit étant contenu dans un dispositif de conditionnement, le procédé comportant les étapes suivantes :
- placer le dispositif de conditionnement dans un four à micro-ondes,
- élever la température du produit en le soumettant à un rayonnement micro-ondes à l'intérieur du four,
- appliquer le produit au moyen d'un applicateur.

L'invention concerne également les dispositifs de conditionnement de tel produit cosmétique aptes à être chauffés dans un four à micro-ondes.

## Description

La présente invention concerne les procédés de maquillage et d'application d'un produit de soin ainsi que les dispositifs utilisés dans la mise en oeuvre de tels procédés.

Il a été proposé d'appliquer des produits cosmétiques après avoir élevé leur température. Ainsi, la demande de brevet français FR 2 376 401 propose de chauffer les shampoings à une température légèrement supérieure à celle du corps humain afin d'obtenir une meilleure efficacité. Pour cela, les doses de shampoing sont placées dans un appareil comportant des résistances chauffantes. Le brevet US 5 775 344 décrit un dispositif de conditionnement et d'application de mascara comportant une résistance chauffante intégrée au récipient.

La demande internationale WO 00/43286 décrit un dispositif comportant des composés qui, lorsque mélangés, produisent une réaction exothermique permettant d'élever la température d'une composition cosmétique.

Le document DE-43,12,278 enseigne la préparation d'une composition cosmétique en réchauffant préalablement la phase grasse au four à micro-ondes avant d'y incorporer l'eau à mélanger avec cette phase grasse pour obtenir ladite composition cosmétique.

L'invention, selon un premier de ses aspects parmi d'autres, a pour objet un procédé pour l'application, notamment sur des fibres kératiniques, d'un produit cosmétique, y compris de soin, comportant des particules de forme allongée telles que des fibres, ce produit étant contenu dans un dispositif de conditionnement, le procédé comportant les étapes suivantes :
- placer le dispositif de conditionnement dans un four à micro-ondes,
- élever la température du produit en le soumettant à un rayonnement micro-ondes à l'intérieur du four,
- appliquer le produit au moyen d'un applicateur.

Par « fibres kératiniques », on entend toute fibre constituée essentiellement de kératine telle que les cheveux, les poils, les cils et les sourcils.

Le terme «particules de forme allongée» englobe notamment des particules non sphériques, comportant au moins un axe d'allongement, par exemple discernables individuellement à l'oeil nu. Il couvre également les fibres destinées par exemple à allonger les cils et/ou à produire des effets esthétiques, notamment de couleur et/ou de brillance. Les paillettes constituent un autre exemple de ces particules de forme allongée. Les micas et les nacres peuvent constituer d'autres exemples encore de ces particules de forme allongée.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150. En particulier, les fibres ont une longueur allant de 1 µm à 10 mm, de préférence de 0,1 mm à 5 mm et mieux de 0,3 mm à 3 mm. Par exemple, les fibres peuvent présenter une section circulaire ou polygonale.

Les fibres peuvent être présentes dans la composition selon l'invention en une teneur allant de 0,1 à 10% en poids, mieux de 0,5 à 5% en poids par rapport au poids total de la composition.

Le produit peut comporter des fibres seulement, des paillettes seulement, ou un mélange des deux. Le produit peut comporter des paillettes dont une plus grande dimension moyenne est comprise par exemple entre 50 µm et 1,5 mm. Le produit peut comporter au moins 0,2 % en poids de ces particules, par exemple des fibres et/ou des paillettes. Le produit peut être destiné à l'application sur les cils et/ou les sourcils. Le produit peut être une composition de revêtement des fibres kératiniques tel qu'un mascara, et les particules sont alors dispersées dans cette composition.

Les fibres peuvent par exemple être choisies parmi les fibres rigides ou non rigides. Elles peuvent être d'origine synthétique ou naturelle, minérale ou organique. A titre d'exemple, on peut citer les fibres non rigides telles que les fibres de polyamide (Nylon®), de cellulose (ou de rayonne), de polyéthylène ou encore les fibres rigides telles que les fibres de polyimide-amide comme celles vendues ou de poly-(p-phénylène-téréphtalamide) (ou d'aramide). On peut encore utiliser des fibres d'origine animale ou végétale comme des fibres de coton ou de lin, ou de synthèse, en particulier réalisées à partir d'un polymère thermoplastique, ou en verre.

De préférence, le produit est chauffé en soumettant le dispositif de conditionnement à un rayonnement micro-ondes à l'intérieur d'un four à micro-ondes. Dans la mesure où ces particules ont une nature différente du produit dans lequel elles sont contenues, leur température peut être plus rapidement élevée que celle du produit. Ainsi, même si le dispositif de conditionnement n'est pas exposé à un rayonnement micro-ondes suffisamment long pour rendre le produit intégralement fluide, l'élévation en température de ces particules fluidifie le produit au moins localement autour de chaque particule. Lors de l'application du produit au moyen de l'applicateur, les particules peuvent alors être orientées relativement à la surface sur laquelle elles sont appliquées. Si les particules sont réparties de manière homogène dans le produit, le produit peut alors être réchauffé et liquéfié en un temps plus court.

Par « four à micro-ondes », on désigne les fours conventionnels utilisés par ailleurs pour réchauffer les aliments et comportant une enceinte à l'intérieur de laquelle un objet à chauffer peut être exposé à une énergie électromagnétique.

Par rapport à un chauffage par exposition à une source de rayonnement infrarouge, l'utilisation d'un four à micro-ondes permet d'obtenir une élévation en température rapide quelles que soient les caractéristiques de conduction de la chaleur des parois du récipient contenant le produit.

Le produit peut ainsi être rapidement et aisément porté à la température souhaitée sans pour autant avoir à incorporer dans le dispositif de conditionnement une résistance chauffante. La température au coeur de la masse de produit est élevée rapidement. L'élévation en température du produit réchauffe progressivement les parois du récipient qui sont à son contact. Ce produit est de plus chauffé de manière homogène par ce type de rayonnement. Le chauffage du produit peut également permettre de le rendre, éventuellement de nouveau, homogène. Il n'y a pas de séparation des phases chimiques du produit du fait que l'élévation en température est homogène.

Le chauffage du produit peut viser par exemple à favoriser son étalement, ou sa tenue sur une zone à enduire, par exemple les cils, les cheveux, la peau ou les muqueuses, ou encore améliorer la mise en plis des cheveux. Notamment le chauffage du produit peut en améliorer ses propriétés dermatologiques, et ou améliorer ses effets allongeant, recourbant, de brillance, de matification, de douceur, et ou d'antirides. Ce chauffage peut présenter un intérêt dans le cadre de l'aromathérapie, pour favoriser les effets des arômes et des huiles essentielles appliquées. L'application d'un produit cosmétique chaud peut également simuler un effet de sauna ou de hammam sur la peau dans le cadre d'une application topique. Elle peut favoriser la pénétration d'un actif de ce produit dans la peau, les muqueuses ou les fibres kératiniques et exercer une action locale sur la circulation sanguine par exemple.

Par exemple, le produit peut procurer au moins deux types d'effets en fonction de la température à laquelle il est utilisé. Avantageusement, le produit présente des propriétés qui permettent une application d'une part à chaud et d'autre part à froid, notamment à température ambiante. Par exemple, à froid, l'application du produit sur les fibres kératiniques conduit à un maquillage chargé, alors qu'à chaud l'application de ce produit sur les fibres kératiniques conduit à un maquillage plus allongeant et à un meilleur gainage des fibres. Cela peut permettre à l'utilisateur d'adapter au mieux les propriétés d'un produit au type d'application ou de maquillage, ou de soin souhaité.

Par exemple, une application du produit à chaud permet de l'étaler sur une couche plus fine pour obtenir un maquillage moins couvrant, et une application de ce même produit à température ambiante permet un maquillage matifiant. En effet, le produit étant plus fluide à chaud, on peut couvrir une plus grande surface avec une quantité moins importante de produit, d'où l'atténuation du maquillage qui peut être obtenue à chaud.

De même, une composition comportant les particules étant rendue plus fluide à chaud qu'à température ambiante, l'homogénéisation de la dispersion de ces particules dans la composition en est plus aisée. Par ailleurs, lors de l'application, les particules étant plus mobiles dans la composition, ces particules peuvent être déposées de manière homogène sur les cils, les cheveux ou la peau.

En particulier, lorsque le produit est une composition apte à recouvrir des fibres kératiniques faiblement fluide, voir pâteuse, à température ambiante, l'application « à chaud » de cette composition au moyen d'un applicateur, tel qu'une brosse ou un peigne, sur lesdites fibres kératiniques favorise la disposition des particules de forme allongée sur ces fibres kératiniques. En effet, la composition étant plus fluide du fait de son chauffage, les particules de forme allongée se disposent plus facilement selon le mouvement qui leur est impliqué par l'applicateur. Les particules comportant un axe d'allongement se disposent alors le long de chacun des cils pour en renforcer leur diamètre et éventuellement pour en prolonger leur longueur.

Les propriétés rhéologiques du produit peuvent être modifiées par la température, notamment la viscosité, la tension superficielle, la composition structurelle, et la thixotropie, le cas échéant. Le chauffage peut alors faciliter le prélèvement du produit. Le chauffage des produits diminue leur viscosité. Ils s'étalent alors plus facilement. De préférence, la composition est thermoréversible, et après chauffage retrouve ses propriétés initiales quasi identiques à celles présentées avant le chauffage.

Par exemple, dans le cas des préparations extemporanées comportant au moins deux composants, par exemple deux liquides ou une poudre et un liquide, par exemple des préparations capillaires extemporanées, le chauffage peut accélérer le mélange des composants et/ou la dissolution d'un composant dans un autre.

Le chauffage peut aussi permettre de re-liquéfier le produit ayant séché sur les parois du récipient, et de le faire couler dans le fond de ce récipient afin d'en faciliter la vidange.

Par le biais de ce chauffage aux micro-ondes, on peut obtenir une purification bactérienne, voire une pasteurisation, du produit contenu dans le récipient. Cette pasteurisation peut être effectuée plusieurs fois durant la vie du produit. Ce chauffage permet d'envisager des formulations de produit comportant moins de conservateurs.

De préférence, le produit contient de l'eau ou toute autre substance absorbant le rayonnement émis par les fours à micro-ondes, et s'échauffant sous l'action des micro-ondes.

Un avantage supplémentaire lié à l'utilisation de micro-ondes pour élever la température du produit réside dans la possibilité de réaliser le dispositif de conditionnement avec un isolant thermique, perméable aux micro-ondes, permettant une conservation de la chaleur du produit au cours de l'utilisation, et ou permettant d'offrir une surface de préhension moins chaude que la température du produit contenu dans le récipient.

Le produit peut être chauffé dans le four à micro-ondes de façon à ce que sa température soit par exemple comprise entre 30 °C et 80 °C.

La durée pendant laquelle le produit est exposé au rayonnement micro-ondes peut être comprise par exemple entre 1 et 60 secondes, mieux entre 2 et 50 secondes, voire entre 3 et 25 secondes étant par exemple voisine de 5 secondes. Le chauffage du produit peut être fractionné en plusieurs étapes de chauffe entrecoupée de pauses. Ces pauses permettent éventuellement à l'utilisatrice d'assurer un contrôle de la température atteinte par le produit.

Cette durée dépend notamment de la puissance du four, de la nature du récipient, de la température de départ et de la température à atteindre, de la quantité et de la nature du produit à chauffer. Le dispositif peut comporter un tableau renseignant l'utilisateur sur la durée de chauffage nécessaire en fonction de la puissance du four, par exemple.

Le procédé peut être mis en oeuvre une ou plusieurs fois, selon que le dispositif est à usage unique ou non.

Le dispositif peut comporter un récipient présentant une forme permettant d'accroître l'exposition au rayonnement micro-ondes. Le récipient peut notamment comporter un repère indiquant à l'utilisateur la position dans laquelle doit être placé préférentiellement le récipient dans le four à micro-ondes. Le récipient peut par exemple être agencé de manière à ce que la hauteur de produit soit plus faible dans une position de chauffage que dans une position d'utilisation normale du récipient. La hauteur du produit est mesurée selon une verticale entre le niveau inférieur du produit et son niveau supérieur. La position de chauffage peut par exemple être une position couchée pour tenir compte de la répartition horizontale d'un champ de micro-ondes dans la cavité du four.

Alternativement, la position de chauffage peut être une position surélevée, par exemple obtenue au moyen d'un rehausseur du dispositif de conditionnement, de telle sorte que le dispositif soit soumis à des rayonnements maximum en étant placé dans une zone centrale du volume du four. Ce rehausseur est par exemple posé sur le fond du four, et par coopération avec le dispositif permet de surélever le récipient relativement à ce fond.

Dans le cas des récipients munis d'un embout d'application, en particulier en mousse, ou d'un essoreur, on dispose de préférence ces récipients de telle sorte que leur embout d'application et ou leur essoreur soient respectivement chauffés par contact avec le produit chauffé à coeur sous l'effet du rayonnement des micro-ondes. On dispose le récipient, fermé, "tête en bas" à l'intérieur du four à micro-ondes.

L'embout d'application peut être un peigne par exemple obtenu par injection d'un matériau thermoplastique, ou encore une brosse par exemple obtenue par sur-injection sur des poils destinés à former des éléments d'application de cette brosse. L'embout d'application peut être floqué. De préférence, l'embout d'application est réalisé dans un matériau pouvant être chauffé dans un four à au micro-ondes.

Le récipient peut comporter une paroi agencée pour permettre de le poser de manière stable en position couchée, par exemple une paroi ayant une section transversale prismatique offrant au moins une surface plane, plutôt qu'une section transversale circulaire.

Le dispositif de conditionnement peut comporter un signe renseignant l'utilisateur sur la possibilité de le placer dans un four à micro-ondes. Ce signe peut par exemple être constituée d'une appellation telle que "microwave advanced technology".

L'invention a encore pour objet, selon un autre de ses aspects, indépendant ou pouvant se combiner avec ce qui précède, un dispositif de conditionnement comportant un applicateur et un récipient contenant un produit cosmétique, y compris de soin, ce produit comportant des particules de forme allongée et le dispositif étant apte à être chauffé dans un four à micro-ondes. Par exemple, l'applicateur est préalablement détaché du récipient lorsque le produit est soumis au rayonnement micro-ondes.

Par exemple l'applicateur peut être maintenu hors du four lors de l'élévation en température du produit, notamment lorsque l'applicateur est incompatible avec un chauffage au four à micro-ondes. A contrario, l'applicateur peut aussi, dans les cas où cela est rendu possible, être disposé dans le four à micro-ondes en vue de le soumettre à un tel rayonnement pour par exemple augmenter la température de l'applicateur en surface, et en particulier au niveau de sa surface d'application.

De préférence le dispositif comporte au moins un indicateur agencé pour délivrer une information relative à la température du produit.

Le dispositif de conditionnement ou un sur-emballage signalent par exemple cette possibilité de chauffer le dispositif de conditionnement dans un four à micro-ondes. Un tel indicateur peut renseigner l'utilisateur sur la température du produit à sa sortie du four à micro-ondes, afin de lui permettre d'utiliser le produit dans de bonnes conditions, à la température qu'il souhaite, et notamment pour lui éviter de se brûler.

Le dispositif étant agencé pour permettre de chauffer le produit dans un four à micro-ondes, il est avantageusement dépourvu de métal, par exemple de pièce métallique ou de revêtement métallisé ou électriquement conducteur, étant de préférence réalisé uniquement avec des matériaux compatibles avec une utilisation dans un four à micro-ondes. Après chauffage au four à micro-ondes, la température extérieure du récipient est généralement inférieure à la température intérieure du produit.

L'indicateur utilisé peut notamment permettre d'avertir l'utilisateur lorsque la température du produit est supérieure à au moins une valeur prédéfinie. Cet indicateur peut être solidaire du récipient contenant le produit. Dans le cas où le dispositif comporterait un organe de fermeture du récipient, l'indicateur peut être solidaire de cet organe de fermeture. Le dispositif peut encore comporter un applicateur et l'indicateur être alors solidaire de cet applicateur, cet indicateur peut ainsi être directement au contact du produit, pendant que le dispositif est placé au four à micro-ondes. Dans le cas où le dispositif comporterait un récipient à fond rapporté, l'indicateur peut être solidaire de ce fond ou être retenu par lui sur le récipient. Le fond est par exemple claqué ou soudé sur le récipient. Le cas échéant, l'indicateur peut également être présenté par le rehausseur.

L'indicateur sensible à la température peut être disposé de diverses manières sur le dispositif. L'indicateur peut être amovible ou fixé à demeure sur le dispositif de conditionnement. Dans le cas où l'indicateur est amovible, il se présente par exemple sous la forme d'une bague élastomère comportant des pigments thermochromiques, pouvant être coulissée et retenue sur un pourtour extérieur du récipient. L'indicateur peut par exemple comporter au moins un support flexible fixé, par exemple par collage ou soudure, sur le dispositif. L'indicateur peut se présenter par exemple sous la forme d'une pastille ou d'une étiquette adhésive collée sur une paroi du dispositif, par exemple une paroi du récipient ou d'un organe de fermeture du récipient.

L'indicateur peut encore être réalisé par une impression, ou sérigraphie, sur le dispositif d'une encre comportant un pigment thermochromique. L'indicateur peut encore être formé par une incorporation d'un pigment thermochromique dans la matière d'une partie au moins du dispositif, par exemple la matière d'une partie au moins du récipient, d'une partie au moins d'un organe de fermeture du récipient, d'une partie au moins d'un fond rapporté le cas échéant, ou d'une partie au moins de l'applicateur, par exemple une partie au moins d'un élément d'application ou d'une tige reliant l'élément d'application à un organe de préhension, lequel peut servir le cas échéant à fermer le récipient.

Le dispositif comportant de tels pigments thermochromiques peut par exemple être obtenu par bi-injection. Dans ce cas, un hublot peut être réalisé dans une paroi du récipient et comporte lesdits pigments.

L'indicateur peut comporter tout matériau changeant d'aspect avec la température et par exemple des cristaux liquides cholestériques, éventuellement encapsulés, et de préférence un matériau compatible avec le passage du dispositif dans un four à micro-ondes. A titre d'exemple de matériaux changeant d'aspect avec la température, on pourra se référer par exemple à la demande EP 1 191 317 A1 ou au brevet US 5 786 578.

L'indicateur sensible à la température peut par exemple changer d'aspect, notamment de couleur, avec la température, en passant d'une couleur à une autre lorsqu'une température prédéfinie de virage est franchie. L'indicateur peut encore présenter une transparence qui dépend de la température, et devenir transparent ou opaque à partir d'une certaine température. De préférence, l'indicateur change d'aspect de façon réversible avec la température, c'est-à-dire qu'il reprend son aspect initial lorsque le dispositif revient à sa température initiale. L'indicateur peut encore présenter une saturation de sa coloration qui est fonction de la température.

On choisit l'indicateur en fonction de sa température de virage, et en fonction de l'épaisseur de la paroi sur lequel il est appliqué pour tenir compte, le cas échéant de l'inertie thermique de cette paroi, de son coefficient de transmission de la chaleur, de sa résistance thermique, et de son coefficient d'absorption de la chaleur. Dans ce cas, on choisit un indicateur changeant d'état à une température inférieure à la température à laquelle on souhaite réchauffer le produit, pour tenir compte de cet écart de température qui peut exister entre le produit et l'indicateur lorsque ce dernier n'est pas directement au contact du produit mais est séparé de celui-ci par la paroi du récipient.

On peut nécessiter deux types d'indications. Premièrement, l'utilisatrice peut vouloir savoir si la température atteinte par le produit n'est pas dangereuse, c'est à dire qu'elle est inférieure à une première température Tₘₐₓ, ou T₁, par exemple de l'ordre de 50°C. Deuxièmement, l'utilisatrice peut également vouloir savoir si la température du produit a bien été élevée par le procédé de chauffage, et qu'elle est bien supérieure à une deuxième température T_{chaud}, ou T₂, par exemple de l'ordre de 30°C pour garantir une application "à chaud" de ce produit. Quand la température est comprise entre T_{chaud} et Tₘₐₓ, on est dans les bonnes conditions pour une utilisation du produit "à chaud".

Dans un premier mode de réalisation, on prévoit de disposer deux indicateurs distincts sur le dispositif, chacun de ces indicateurs ayant une température de virage différente. Un premier indicateur ayant une température de virage permettant de connaître le dépassement de la température T₁, et un deuxième indicateur ayant une température de virage permettant de connaître le dépassement de la température T₂. Les températures de virage sont par exemple choisies inférieure de quelques °C à T₁ et T₂ du fait de la résistance thermique des parois entre l'indicateur et le produit.

Dans un deuxième mode de réalisation, on prévoit un indicateur de température disposé sur une paroi du dispositif présentant au moins deux épaisseurs différentes. Ainsi en jouant sur la résistance thermique de cette paroi, liée à son épaisseur, on peut voir le même indicateur ayant viré dans une première zone, et resté inchangé dans une deuxième zone. Lorsque seule la zone de faible épaisseur a son indicateur qui a changé de couleur, et que ce même indicateur sur la zone de plus forte épaisseur est resté inchangé, alors l'utilisatrice peut saisir le récipient et appliquer le produit. De préférence les zones de plus forte épaisseur constituent des zones de préhension car elles sont de fait moins chaudes. Dans ce cas, pour que l'indicateur soit fiable, il faut maîtriser avec précision l'épaisseur de la paroi du récipient au niveau de laquelle l'indicateur est fixé.

Pour obtenir un tel récipient avec une épaisseur de paroi variable, on prévoit par exemple des stries horizontales, verticales ou hélicoïdale dépassant d'un pourtour extérieur du récipient. En variante, le récipient présente, selon une section transversale un pourtour intérieur circulaire, alors que son pourtour extérieur peut présenter tout type de forme exceptée celle d'une forme également circulaire dont le centre serait superposé à celui du pourtour intérieur. Par exemple le pourtour extérieur, selon cette même section transversale, peut présenter une forme circulaire excentrée, ovoïdale, triangle, rectangle, ou de n'importe quel type de polygones.

Le dispositif peut également comporter un applicateur du produit contenu dans le récipient; Cet applicateur peut ou non être soumis à un rayonnement micro-ondes. Dans le cas où l'applicateur ne peut pas être ainsi chauffé, on le dispose dans un support pendant que le réservoir est soumis au rayonnement micro-ondes. Par exemple, ce support est prévu pour être fixé sur le réservoir dans une position de stockage, hors période de chauffe du réservoir.

Cet applicateur est de préférence solidaire d'un bouchon permettant d'obturer le récipient. L'applicateur peut être réalisé par moulage d'un seul tenant avec le bouchon, ou bien y être rapporté et retenu par surmoulage ou collage.

Le dispositif peut comporter au moins une soupape de sécurité permettant d'éviter l'accumulation d'une pression excessive à l'intérieur du dispositif si celui-ci est exposé par inadvertance à un rayonnement micro-ondes pendant une durée trop longue.

Le dispositif peut également comporter un organe anti-projection permettant de réduire le risque de projection de produit à l'ouverture du dispositif sous l'effet d'une surpression créée par le chauffage du produit, par exemple à cause de la dilatation de l'air. Un tel organe peut être actionnable ou non par l'utilisateur préalablement à l'ouverture. Ainsi, dans une réalisation, l'organe anti-projection comporte une partie sur laquelle l'utilisateur peut appuyer pour équilibrer les pressions entre l'intérieur du récipient et l'extérieur, préalablement à l'ouverture du récipient.

En variante, l'organe anti-projection remplit automatiquement sa fonction à l'ouverture du récipient ou lors de l'extraction de l'applicateur. Cet organe anti-projection peut notamment servir en outre à l'essorage de l'élément d'application. Dans une autre variante encore, c'est l'applicateur lui-même qui peut permettre de réduire le risque de projection de produit à l'ouverture, par exemple en formant un écran vis-à-vis d'éventuelles projections de produit.

Le dispositif peut également comporter un organe réducteur d'écoulement servant par exemple à réduire le risque de perte de produit en cas de renversement accidentel du récipient, notamment si la fluidité du produit a fortement augmenté avec la température.

L'invention a encore pour objet, indépendamment ou en combinaison avec ce qui précède, une méthode pour promouvoir la vente d'un dispositif de conditionnement contenant un produit cosmétique, y compris de soin, à l'exclusion des cires dépilatoires, dans laquelle on fait état de la possibilité de placer le dispositif dans un four à micro-ondes pour élever la température du produit afin par exemple de modifier des propriétés de celui-ci, notamment sa rhéologie. Ce produit est par exemple un produit de soin ou de maquillage tel qu'un fond de teint ou un mascara.

L'invention a encore pour objet, indépendamment ou en combinaison avec ce qui précède, une méthode pour promouvoir la vente d'un dispositif de conditionnement et d'application contenant un produit cosmétique, dans laquelle on fait état de la possibilité d'obtenir deux effets de maquillage différents selon que l'on utilise le produit à chaud ou à froid.

La promotion du produit pourra se faire par n'importe quel canal de communication. Elle pourra notamment être faite par un vendeur, directement sur le point de vente, par la radio ou la télévision, notamment dans le cadre de spots publicitaires. Elle pourra être faite également par le canal de la presse écrite ou par le biais de tout autre document, en particulier à des fins publicitaires. Elle pourra se faire également par un réseau informatique ou de téléphonie mobile. Elle pourra être faite encore sur le dispositif de conditionnement ou sur un emballage ou une notice explicative qui lui est associée.

L'invention a encore pour objet, selon un autre de ses aspects, indépendamment ou en combinaison avec ce qui précède, un dispositif de conditionnement d'un produit cosmétique, comportant :
- un récipient ayant une paroi réalisée au moins en partie dans un premier matériau,
- un isolant thermique définissant au moins partiellement la surface extérieure du récipient, cet isolant thermique étant par exemple réalisé dans un deuxième matériau ayant une conductivité thermique inférieure à celle du premier matériau,
- un produit cosmétique, y compris de soin, contenu dans le récipient.

L'invention a encore pour objet l'utilisation d'un tel dispositif dans un procédé comportant les étapes suivantes :
- élever la température du produit en le plaçant de préférence dans un four à micro-ondes,
- appliquer le produit au moyen de l'applicateur.

L'invention a encore pour objet, indépendamment ou en combinaison avec ce qui précède, un dispositif comportant :
- un récipient,
- un produit cosmétique, y compris de soin, contenu dans le récipient,
- un applicateur pourvu d'un élément d'application,
- un organe d'essorage agencé pour essorer l'élément d'application à sa sortie du récipient, l'applicateur et l'organe d'essorage étant agencés pour laisser s'échapper l'air à l'ouverture du dispositif en cas de surpression dans le récipient.

L'invention a encore pour objet l'utilisation d'un tel dispositif dans un procédé comportant les étapes suivantes :
- élever la température du produit en plaçant le dispositif dans un four à micro-ondes de préférence,
- appliquer le produit.

L'invention a encore pour objet l'utilisation d'un dispositif comportant :
- un récipient,
- un produit cosmétique contenu dans le récipient,
- un applicateur comportant au moins une partie réalisée dans un matériau ayant une capacité calorifique lui permettant d'emmagasiner de la chaleur,
dans un procédé comportant les étapes suivantes :
- élever la température du produit en plaçant le dispositif dans un four à micro-ondes de préférence,
- appliquer le produit.

La capacité calorifique souhaitée peut être obtenue en utilisant par exemple une céramique ou une matière plastique comportant un pourcentage élevé, par exemple supérieur ou égal à 60 % en poids d'une charge, par exemple minérale, notamment métallique ou encore d'une céramique ou une structure poreuse capable de se charger en profondeur avec le produit, ce dernier emmagasinant alors la chaleur.

L'invention pourra être mieux comprise à la lecture de la description détaillée qui va suivre, d'exemples de mise en oeuvre non limitatifs de celle-ci, ainsi qu'à l'examen du dessin annexé, sur lequel :
- la figure 1 est un schéma en blocs illustrant des étapes d'un exemple de procédé conforme à l'invention,
- les figures 2 à 10 illustrent différentes possibilités de réalisation, parmi d'autres, de l'indicateur sensible à la température,
- les figures 11 à 15 représentent différentes possibilités de réalisation d'une isolation thermique sur le récipient,
- la figure 16 est un exemple de réalisation d'organe anti-projection permettant de laisser échapper une surpression,
- la figure 17 représente isolément en vue de dessous l'organe anti-projection selon la flèche XVII de la figure 16,
- les figures 18 et 19 représentent d'autres exemples de moyens permettant de laisser échapper une surpression,
- la figure 20 représente partiellement, en élévation, un exemple d'applicateur rotatif,
- la figure 21 représente partiellement, en élévation, un exemple de dispositif comportant un applicateur en mousse, solidaire du récipient,
- la figure 22 représente en coupe longitudinale, schématique et partielle, un exemple de dispositif comportant un élément d'application solidaire d'un organe de fermeture du récipient,
- la figure 23 représente isolément un poil surmoulé,
- la figure 24 représente un exemple d'applicateur réalisé dans une matière présentant une capacité calorifique élevée,
- la figure 25 représente de manière schématique un appareil destiné au recourbement des cils et intégrant un élément chauffant,
- la figure 26 illustre un exemple de réalisation d'un récipient ayant une forme offrant une position couchée stable;
- les figures 27 et 28 représentent partiellement et schématiquement des récipients équipés de moyens de sécurité actifs en cas de pression trop élevée dans le récipient;
- la figure 29 représente une vue en coupe longitudinale d'un dispositif selon l'invention tel que le réservoir comporte une paroi latérale 41 d'épaisseur variable longitudinalement;
- les figures 30a, 30b et 30c représentent partiellement et schématiquement des récipients de dispositif selon l'invention munis de stries sur leur pourtour extérieur respectivement longitudinales, transversales ou hélicoïdales modifiant localement l'épaisseur de la paroi latérale 41 ;

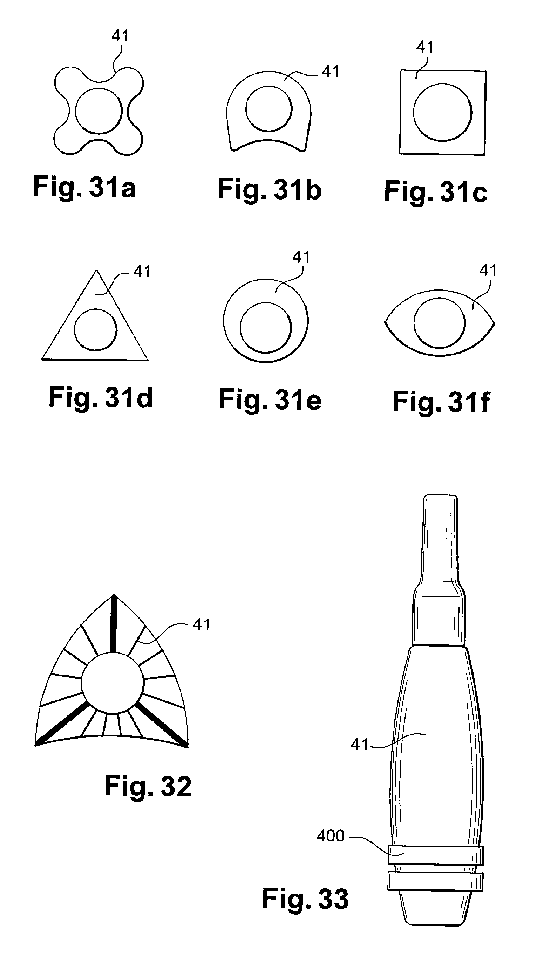

- les figures 31a à 31f représentent des vues en coupe transversale de récipients de dispositifs selon l'invention, pour lesquels l'épaisseur de la paroi latérale 41 du récipient peut être variable au niveau du pourtour extérieur selon ce plan de coupe;
- la figure 32 représente une vue en coupe transversale d'un récipient d'un dispositif selon l'invention comportant une paroi latérale 41 munie de stries longitudinales de largeur variable, ces stries s'étendant radialement relativement à un pourtour intérieur circulaire du réservoir;
- la figure 33 représente un dispositif selon l'invention pour lequel l'indicateur est présenté sous la forme d'une bague 400 coulissée et retenue sur un pourtour extérieur sensiblement cylindrique du réservoir 41;

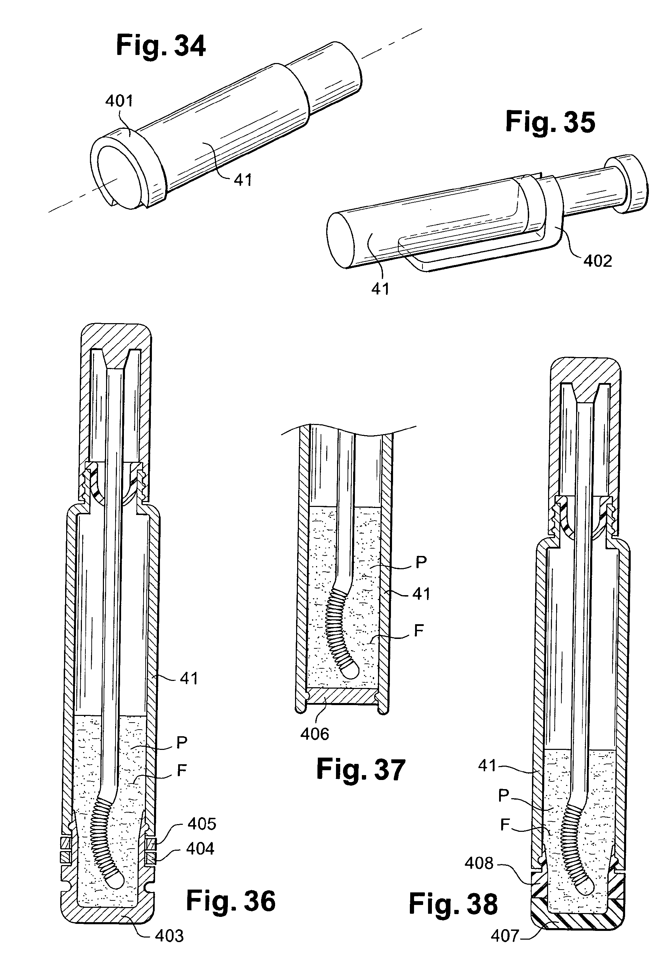

- la figure 34 représente un dispositif selon l'invention pour lequel l'indicateur 401 se présente sous la forme d'un U venant encercler au moins en partie le pourtour extérieur du dispositif, des extrémités libres de ce U venant en appui contre un plan sur lequel le dispositif est allongé. Ainsi l'indicateur en U sert également pour immobiliser le dispositif, en l'occurrence de forme sensiblement cylindrique, dans une position couchée relativement au plan;
- la figure 35 représente un dispositif selon l'invention pour lequel l'indicateur se présente sous la forme d'une agrafe 402 comportant une première portion pour être clipsée sur le pourtour extérieur du dispositif, et une deuxième portion pour venir en appui contre un plan parallèlement auquel le dispositif est allongé. Ainsi cet indicateur en agrafe sert pour immobiliser le dispositif, en l'occurrence de forme sensiblement cylindrique, dans une position couchée relativement au plan, et sert également à le surélever relativement à ce plan;
- la figure 36 représente une vue en coupe longitudinale d'un dispositif selon l'invention comportant un fond 403 rapporté et un applicateur monté dans un capot pour obturer le réservoir. Le fond rapporté est monté sur le récipient de manière étanche, par claquage, mais de manière à également retenir au moins un indicateur 404, ici deux indicateurs 404 et 405 toriques retenus entre le fond 403 et le réservoir 41.
- la figure 37 représente une vue en coupe longitudinale d'un réservoir 41 d'un dispositif selon l'invention, ce réservoir comportant un fond 406 rapporté vissé, tel que ce fond est réalisé dans un matériau comportant des pigments thermochromiques pouvant servir d'indicateur;
- la figure 38 représente une vue en coupe longitudinale d'un dispositif selon l'invention comportant un fond 407 rapporté et un applicateur monté dans un capot pour obturer le réservoir. Le fond rapporté est monté sur le récipient 41 de manière étanche, par vissage, ce fond étant biinjecté de manière à présenter au moins une portion 408 présentant l'indicateur de température, de préférence visible depuis la surface extérieure du fond;
- la figure 39 représente une vue d'un dispositif selon l'invention comportant un hublot 409 permettant de voir au travers le produit P comportant des particules de forme allongée F contenu dans le réservoir; et

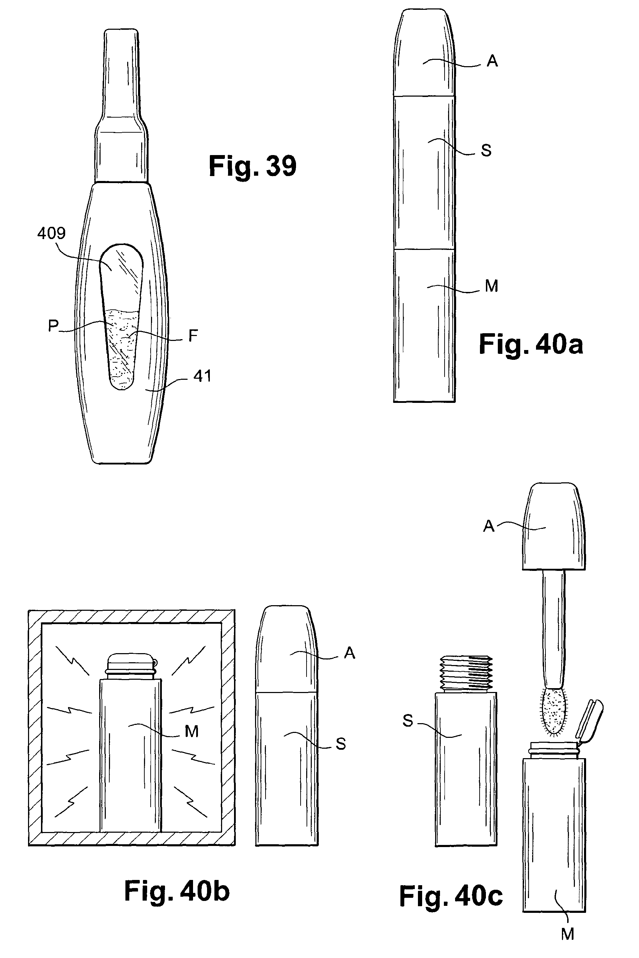

- les figures 40a, 40b et 40c représentent trois étapes successives du procédé selon l'invention dans lequel un récipient M destiné à être chauffé au four à micro-ondes est préalablement désengagé d'un autre support S qui pendant le temps de chauffe du récipient reçoit l'applicateur A, cet applicateur A pouvant être aléatoirement monté sur le récipient M ou le support S. Seulement après chauffage du récipient M au four à micro-ondes, Figure 40b, l'applicateur A est dégagé du support S pour prélever dans le récipient M une dose de produit chauffé. Hors utilisation, comme cela est présenté Figure 40a, le récipient M peut être rendu solidaire du support S dans lequel est rangé l'applicateur A.

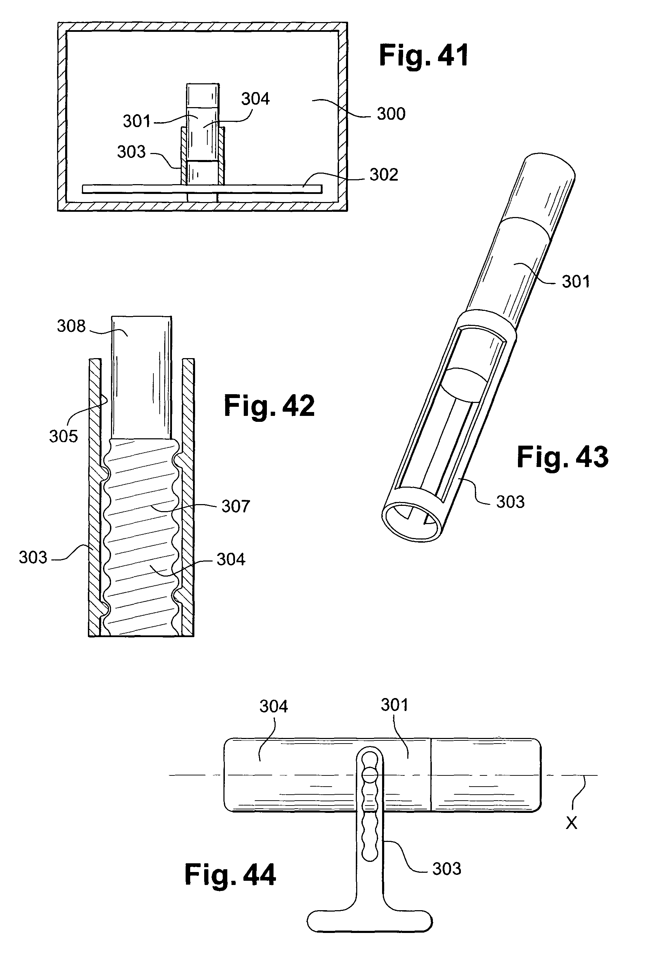

- la figure 41 représente une vue schématique d'une enceinte 300 d'un four à micro-ondes à l'intérieur duquel est disposé un dispositif 301 selon l'invention, ce dispositif contenant un produit cosmétique et étant apte à être chauffé dans un tel four. Par exemple, l'enceinte 300 est équipée d'un plateau tournant 302 sur lequel est placé le dispositif 301. De préférence, le dispositif 301 est disposé au centre du plateau 302. Dans le mode de réalisation représenté, le dispositif 301 est surélevé relativement au plateau 302 au moyen d'un rehausseur 303. Le rehausseur 303 permet de placer le récipient 304 de ce dispositif 301 plus haut relativement au plan formé par le plateau 302. Ainsi, le récipient 304 peut être placé dans une zone centrale de l'enceinte 300, dans laquelle les rayonnements aux micro-ondes sont plus importants.
- Figure 42 : selon un premier mode de réalisation particulier de l'invention, le rehausseur 303 se présente sous la forme d'un manchon muni, au niveau de sa paroi intérieure 305, d'un téton 306. Par exemple, il comporte plusieurs tétons tels que 306. Ce manchon coopère avec un filetage 307prévu ici au niveau du pourtour extérieur du récipient 304. Par exemple, un moyen de fermeture 308 du dispositif 301 dépasse du manchon quel que soit l'engagement du téton 306 avec le filetage 307. Ainsi le consommateur peut facilement placer le dispositif 301 dans une position haute relativement au rehausseur 303 lorsqu'ils sont disposés dans le four. Alternativement, le téton peut être présenté sur le pourtour extérieur du récipient 304 alors que la paroi intérieure 305 du manchon propose un filetage complémentaire.
- Figure 43 : selon un deuxième mode de réalisation particulier de l'invention, le rehausseur 303 se présente sous la forme d'un manchon ajouré que l'on peut translater le long du récipient 304.
- Figure 44 : selon un autre mode de réalisation particulier de l'invention, le rehausseur 303 se présente sous la forme d'un moyen pivotant relativement à un axe orthogonal à un axe d'allongement principal X du dispositif 301. En particulier, le pivot est réalisé au niveau du récipient 304. Par exemple, le rehausseur 303 peut permettre de présenter le dispositif dans une position allongée et à la fois surélevée relativement au plan du plateau 302. L'axe X est alors parallèle à ce plan. En variante, le moyen pivotant peut maintenir l'axe X orthogonal au plan du plateau 302, tout en permettant de surélever le réservoir relativement à ce plan.

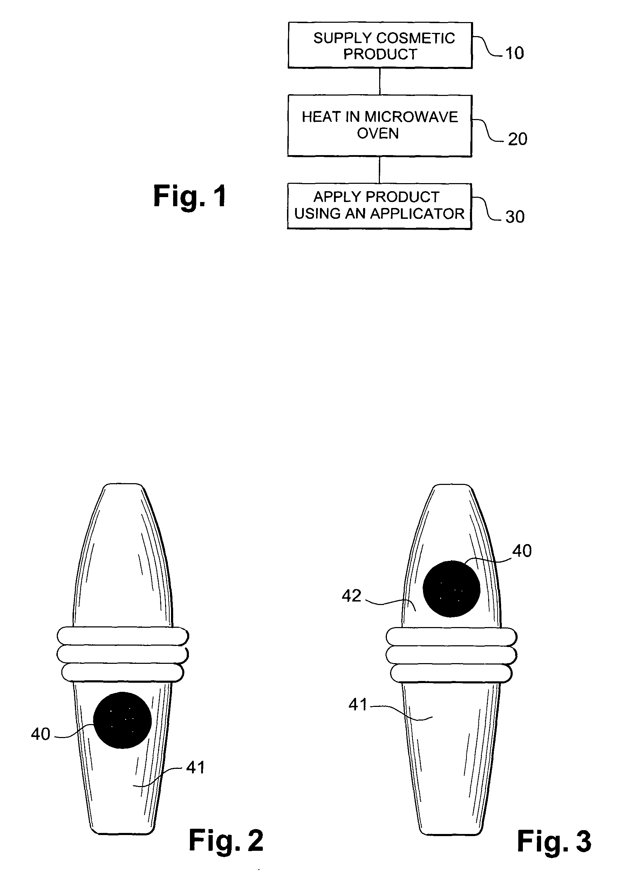

On a illustré sur le schéma en blocs de la figure 1 différentes étapes d'un procédé permettant d'élever la température d'un produit cosmétique, conformément à un premier aspect de l'invention.

Ce procédé comporte une première étape 10 de fourniture du produit cosmétique dans un dispositif de conditionnement, éventuellement muni d'un applicateur. La fourniture du produit cosmétique peut s'effectuer par tout canal de vente, notamment par la vente dans un magasin ou par correspondance, ou par le biais d'un institut de beauté ou d'un salon de coiffure, par exemple.

Le procédé comporte ensuite une étape 20 de chauffage du produit dans un four à micro-ondes. Le dispositif de conditionnement est adapté à être placé dans le four à micro-ondes et ne comporte de préférence aucun élément métallique ou conducteur de l'électricité, susceptible de se détériorer lors de l'exposition au rayonnement micro-ondes ou de détériorer le four utilisé.

La puissance d'émission du rayonnement micro-ondes et la durée d'exposition du produit à ce rayonnement sont choisies en fonction de la température que l'on souhaite atteindre et de la contenance du dispositif de conditionnement. De préférence, on choisit la puissance du four à micro-ondes de telle sorte que la durée pendant laquelle le produit est exposé au rayonnement micro-ondes soit relativement brève, par exemple inférieure à 20 secondes, notamment de l'ordre de quelques secondes.

Ensuite, le dispositif de conditionnement est retiré du four à micro-ondes et l'on procède à l'étape 30 à l'application du produit. Cette application peut s'effectuer par exemple grâce à un applicateur qui peut être monté amovible sur le récipient contenant le produit lorsque celui-ci a été placé dans le four à micro-ondes ou distinct du dispositif de conditionnement. L'application peut également s'effectuer au moyen d'un doigt, par exemple.

Le produit P peut par exemple être un rouge à lèvres, un mascara, un fond de teint ou un produit de soin, cette liste n'étant pas limitative. Le produit P comporte des particules F de forme allongée dispersées dans le produit P.

Un produit préférentiellement destiné à être chauffé est une composition apte à recouvrir des fibres kératiniques.

Par exemple, un produit selon l'invention a la composition suivant :
- Cire d'abeilles 6,00 %
- Cire de paraffine 13,00 %
- Huile de jojoba hydrogénée 2,00 %
- Hydroxyéthyl cellulose 3,00 %
- Stéarate de triéthanolamine 8,00 %
- Pigment noir 5,00 %
- Fibres de Nylon® 2,50 %
- Conservateur qs
- Eau qsp 100,00 %

Le produit peut présenter à température ambiante, c'est-à-dire à 20 °C, des propriétés, notamment rhéologiques, qui permettent une application à cette température, notamment être liquide à température ambiante, ainsi que d'autres propriétés qui permettent aussi une application lorsque la température est plus élevée, par exemple supérieure à 30 °C.

A la fin de la période de chauffage dans le four à micro-ondes, la température du produit à l'intérieur du dispositif de conditionnement peut excéder 50 °C, voire 70 °C. Avantageusement, on équipe le dispositif de conditionnement d'un indicateur sensible à la température, notamment d'un indicateur dont l'aspect, par exemple la couleur, change avec la température. Cet indicateur peut se présenter par exemple sous la forme d'une étiquette ou pastille 40 collée sur la paroi latérale 41 du récipient, comme illustré à la figure 2 ou collée sur un organe 42 de fermeture du récipient, comme illustré à la figure 3. L'organe de fermeture 42 peut éventuellement constituer l'organe de préhension d'un applicateur dont l'élément d'application est présent à l'intérieur du récipient lorsque celui-ci est fermé.

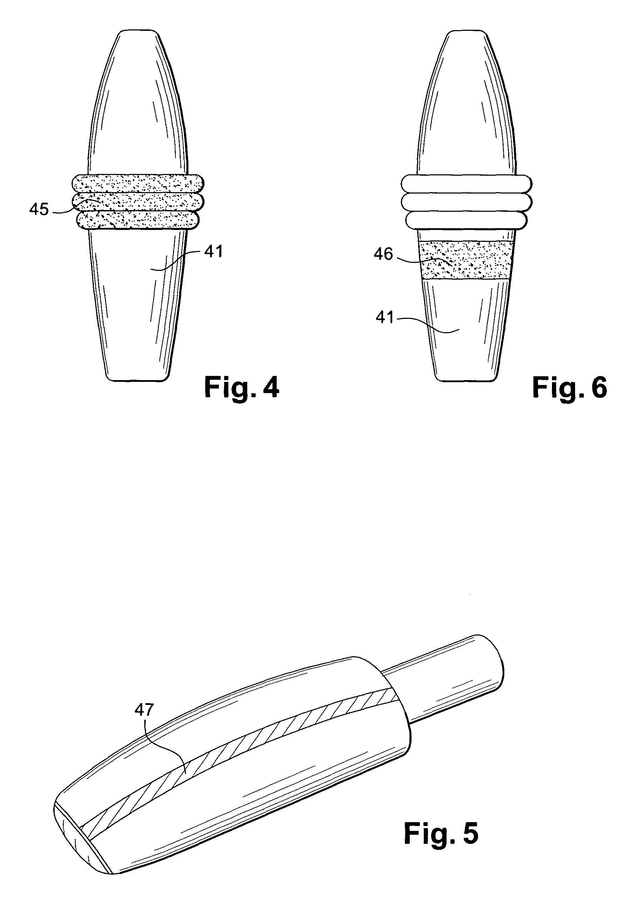

On peut encore réaliser l'indicateur de température en mélangeant à la matière destinée par exemple à former la partie supérieure 45 du récipient un pigment thermochromique. L'indicateur de température est réalisé, dans l'exemple de la figure 5, sous la forme d'une bande 47 réalisée par exemple par bi-injection ou coextrusion avec la paroi du corps du récipient, par exemple dans une matière changeant de couleur avec la température. Cette bande peut servir de hublot si elle est réalisée dans un matériau transparent à au moins certaines températures.

On peut encore déposer par sérigraphie, par exemple sur la paroi latérale 41 du récipient, une bande 46 comme illustré à la figure 6 ou tout autre motif d'une encre comportant un pigment thermochromique.

Dans les exemples des figures 2 à 6, l'indicateur est visible de l'extérieur. On ne sort pas du cadre de l'invention lorsque l'indicateur n'est pas visible de l'extérieur lorsque le récipient est fermé mais le devient soit au moment de l'application soit lorsque l'utilisateur exerce une action particulière, en extrayant du récipient l'indicateur par exemple.

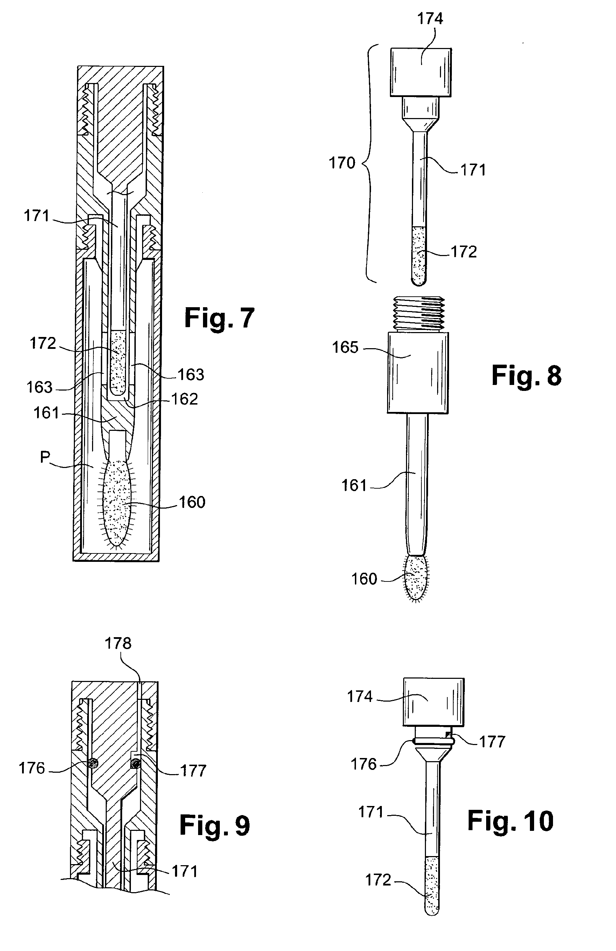

Sur les figures 7 et 8, on a représenté un dispositif ayant un récipient contenant le produit P et un applicateur comportant un élément d'application 160 fixé à l'extrémité d'une tige 161 comprenant un logement 162 dans lequel peut pénétrer un indicateur de température 170. Ce dernier comporte une tige 171 dont l'extrémité 172 change par exemple de couleur avec la température. Des ouvertures 163 peuvent, le cas échéant, être réalisées dans la tige 161 pour permettre au produit P de venir en contact avec l'indicateur de température 170. Dans l'exemple considéré, la tige 171 se raccorde, à son extrémité supérieure, à une partie filetée 174 qui peut se visser sur l'organe de préhension 165 de l'applicateur.

Le cas échéant, comme illustré aux figures 9 et 10, l'indicateur de température peut être fixé sur le dispositif de manière à servir également de soupape de sécurité en cas de pression trop élevée dans le récipient.

La tige 171 de l'indicateur de température peut par exemple porter un joint torique 176 qui en l'absence de surpression assure une fermeture étanche. Un évidement 177 est réalisé dans la tige 171 et en cas de surpression le joint torique 176 se déforme localement en remontant dans cet évidement 177, ce qui permet à l'air sous pression de s'échapper.

Un orifice 178 peut être réalisé, le cas échéant, dans la partie supérieure 178 de l'indicateur de température pour faciliter la sortie de l'air.

L'indicateur de température peut être réalisé par tout moyen connu, notamment à l'aide de tout pigment thermochromique connu ou tout autre matériau changeant de couleur avec la température et compatible de préférence avec la mise en place du dispositif de conditionnement dans un four à micro-ondes pour chauffer le produit.

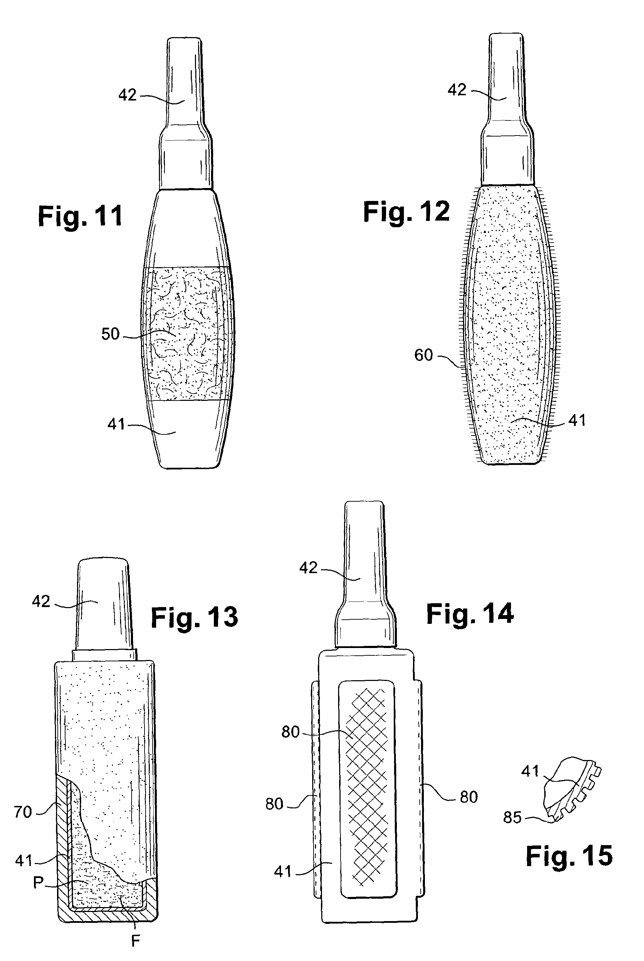

Conformément à un autre aspect de l'invention, le dispositif de conditionnement peut comporter un isolant thermique 50, comme illustré à la figure 11, lequel peut recouvrir par exemple une partie de la paroi latérale 41 du récipient contenant le produit. Cet isolant 50 peut par exemple se présenter sous la forme d'un manchon en un matériau thermiquement moins conducteur que celui servant à réaliser la paroi latérale 41 du récipient, ce manchon étant fixé dans une gorge annulaire réalisée sur le récipient. Un tel isolant 50 permet une préhension plus confortable du récipient lorsque celui-ci sort du four à micro-ondes et que la température du produit avoisine 70 °C par exemple.

L'isolant 50 peut être réalisé par exemple dans une mousse de polyuréthane ou de polyéthylène. L'isolant peut aussi être réalisé différemment et par exemple sous la forme d'un revêtement de flocage 60 couvrant au moins une partie de la paroi latérale 41 du récipient, comme illustré à la figure 12. On peut encore réaliser l'isolant sous la forme d'un étui 70, comme représenté à la figure 13, cet étui 70 ayant par exemple une forme générale de doigt de gant permettant d'y insérer le récipient. On peut ainsi réaliser l'isolant sous la forme de bandes 80 ou autres éléments rapportés, par exemple par collage, sur la paroi latérale 41 du récipient, comme illustré sur la figure 14. Le récipient peut encore être réalisé, par exemple, avec des ailettes 85, comme illustré à la figure 15.

Le dispositif de conditionnement peut être agencé, selon un autre aspect de l'invention, pour permettre à de l'air en surpression à l'intérieur du récipient suite au chauffage du produit de s'échapper à l'ouverture de celui-ci, tout en limitant les risques de projection ou de fuite de produit.

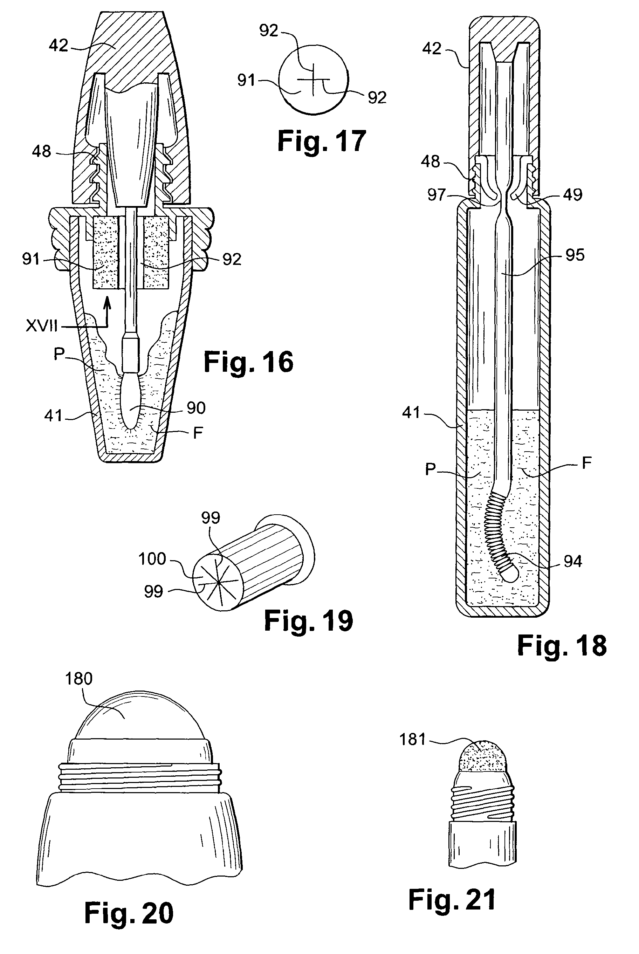

Lorsque le dispositif sert également à l'application du produit et comporte, comme illustré à la figure 16, un applicateur comprenant un élément d'application 90 et un organe d'essorage 91 pour essorer l'élément d'application à sa sortie du récipient, on réalise avantageusement l'organe d'essorage 91 de manière à permettre à une surpression présente à l'intérieur du récipient après le chauffage du produit P de s'échapper progressivement dès l'ouverture du récipient.

L'organe d'essorage 91 peut par exemple se présenter sous la forme d'un bloc de mousse pourvu d'un passage pour l'élément d'application 90, ce passage comportant au moins une fente et par exemple deux fentes 92 dans l'exemple illustré à la figure 17. Ces fentes 92 permettent à l'air sous pression de s'échapper dès que l'organe de fermeture 42 est dévissé suffisamment du col 48 du récipient, tout en limitant le risque de projection de produit.

Sur la figure 17, on voit que l'élément d'application peut se présenter sous la forme d'un applicateur floqué, cet applicateur comportant par exemple un corps en élastomère ou en matière plastique, recouvert à sa surface d'un revêtement de flocage. L'applicateur peut encore ne pas comporter de flocage. L'applicateur peut ainsi être réalisé par exemple par moulage de matière plastique avec une forme particulière, notamment une forme permettant le peignage des cils ou des sourcils.

Sur la figure 18, on a représenté un élément d'application 94 constitué par un peigne, celui-ci étant fixé à une extrémité d'une tige 95 reliée à un organe de préhension qui constitue également un organe de fermeture 42 du récipient.

L'organe d'essorage et anti-projection se présente dans l'exemple considéré sous la forme d'une pièce en élastomère fixée dans le col 48 du récipient et pourvue à son extrémité inférieure d'une lèvre d'essorage 49 définissant une ouverture, circulaire par exemple.

La tige 95 peut présenter une gorge 97 permettant à l'air sous pression de s'échapper lors de l'ouverture du récipient. Cette gorge 97 peut être annulaire ou s'étendre sur une fraction seulement de la circonférence de la tige.

On peut encore réaliser l'organe d'essorage avec une pluralité de fentes radiales 99, comme illustré à la figure 19, ces fentes définissant entre elles des secteurs 100 susceptibles de se déformer sous la pression de l'air à l'intérieur du récipient pour laisser celui-ci s'échapper. On peut encore utiliser d'autres moyens pour réduire le risque de projection de produit en cas de surpression dans le récipient.

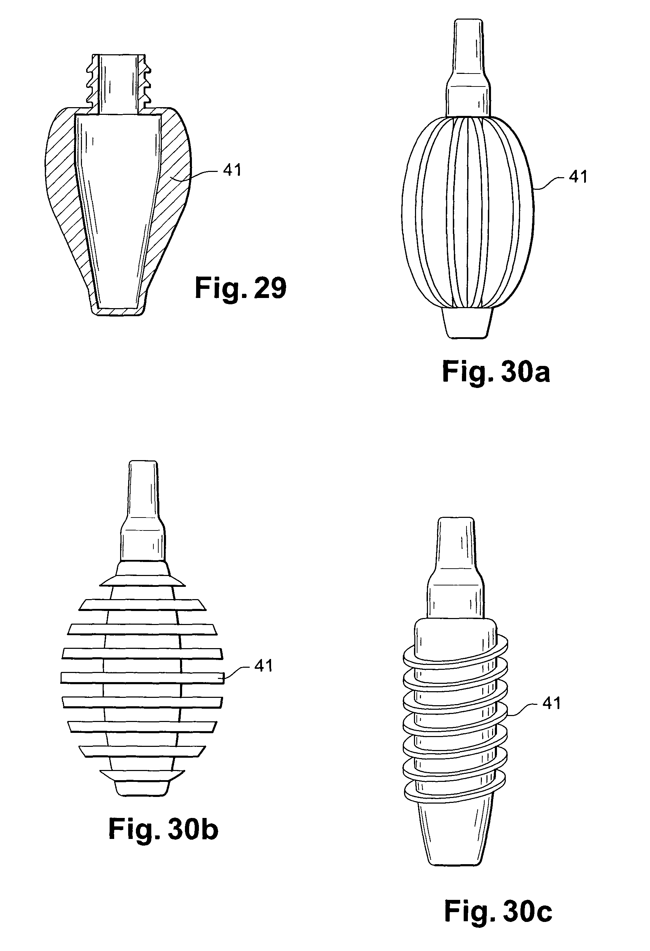

Sur la figure 30, on a représenté partiellement un dispositif dans lequel l'applicateur comporte un élément d'application rotatif tel qu'une bille 180 par exemple. Cette bille 180 permet d'appliquer le produit tout en réduisant le risque de projection de produit lorsque le capot de fermeture, non représenté, est enlevé.

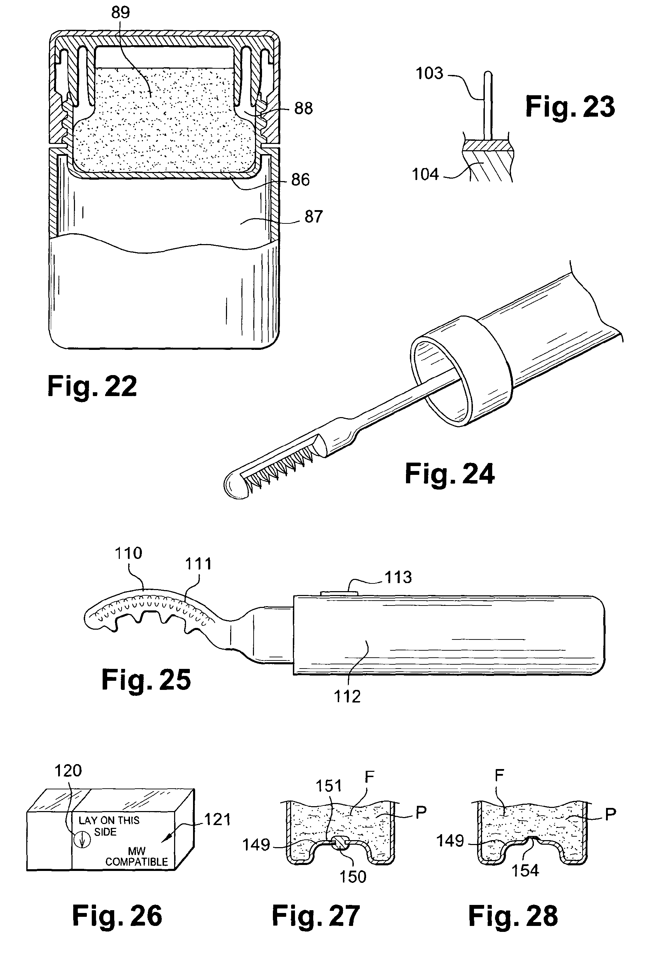

L'élément d'application rotatif peut être remplacé, par exemple, par un élément d'application non rotatif, fixé à demeure sur le récipient, tel qu'une mousse 181 comme illustré sur la figure 21. On peut encore prévoir par exemple entre un espace 87 du récipient contenant le produit et une ouverture 88 par laquelle le produit est prélevé une paroi ajourée 86, comme illustré à la figure 22. Le cas échéant, cette paroi 86 peut servir de siège à un élément d'application 89.

D'une manière générale, lorsqu'un rayonnement micro-ondes est utilisé pour élever la température du produit P, le dispositif de conditionnement est dépourvu d'élément métallique et l'applicateur est réalisé sans métal. On peut ainsi, dans le cas où l'on cherche à utiliser une brosse, réaliser celle-ci avec des poils 103 surinjectés sur un support 104, comme illustré sur la figure 23, les poils et le support étant par exemple réalisés dans des matières thermoplastiques différentes.

Conformément à un autre aspect de l'invention, on peut réaliser l'élément d'application de manière à ce que celui-ci présente une inertie thermique suffisamment élevée pour que le produit présent sur l'élément d'application ne se refroidisse pas trop vite. On peut ainsi réaliser l'élément d'application, lequel est par exemple un peigne comme représenté à la figure 24, dans une matière plastique comportant une proportion importante d'une charge minérale ou autre lui conférant une capacité thermique élevée.

On peut ainsi par exemple réaliser l'élément d'application par moulage d'une matière thermoplastique ou thermodurcissable comportant une charge d'un composé tel que du bronze ou un oxyde d'aluminium. On peut notamment réaliser l'élément d'application par moulage d'un mélange comportant 60 % en poids d'oxyde d'aluminium et le reste de polyamide ou de polypropylène. On peut encore, ceci n'étant qu'un autre exemple non limitatif, réaliser l'élément d'application par moulage d'un mélange comportant 40 % en poids de polypropylène et 60 % d'une céramique.

On a représenté sur la figure 25 un dispositif servant au recourbement des cils, comportant un élément 110 à capacité calorifique élevée, destiné à être amené au contact des cils pour recourber ceux-ci, incorporant une résistance chauffante 111, laquelle est reliée par un circuit électrique non représenté à une source d'énergie électrique contenue dans une poignée 112, un interrupteur 113 permettant d'alimenter la résistance 111. Ce dispositif peut comporter un système thermostatique permettant d'interrompre le passage du courant lorsque l'élément 110 atteint une température suffisante. Ce dispositif applicateur est détaché du réservoir et n'est pas soumis à des rayonnements micro-ondes. Il est utilisé en complément du chauffage du produit.

Le fait que l'inertie thermique de l'élément 110 soit relativement grande grâce par exemple à l'utilisation d'une matière plastique chargée peut permettre de réduire la consommation électrique du dispositif. On peut également accroître l'inertie thermique de l'élément d'application en utilisant, pour réaliser celui-ci, une matière capable de se charger en profondeur avec le produit à appliquer, par exemple une matière poreuse telle qu'une mousse ou un fritté.

Par ailleurs, on peut avantageusement réaliser le récipient avec une forme permettant de le positionner à plat sans qu'il roule dans le four à micro-ondes, afin de tirer profit de la répartition du champ de micro-ondes dans le four. A titre d'exemple, on a représenté à la figure 26 un récipient de section transversale carrée, pouvant être couché sur l'un de ses côtés. On voit sur cette figure que le récipient peut comporter, par exemple, un repère 120 renseignant l'utilisateur sur la façon la plus adaptée de disposer le récipient dans le four à micro-ondes. Le récipient peut comporter également un signe 121 indiquant la possibilité de placer le dispositif dans un four à micro-ondes.

Pour permettre au produit et à l'air de s'échapper en cas de présence accidentelle trop longue dans le four à micro-ondes en fonctionnement, une soupape de sécurité peut être prévue sur le récipient. A titre d'exemple, on a représenté partiellement à la figure 27 en coupe axiale un récipient dont le fond 149 est pourvu d'un obturateur 150 formant soupape de sécurité. Cet obturateur 150 est constitué par exemple par un bouchon en élastomère fixé dans un trou 151 de la paroi de fond 149, capable d'être éjecté en cas de pression trop forte dans le récipient. La soupape de sécurité pourrait être réalisée, en variante, par un amincissement 154 de la paroi de fond, comme illustré à la figure 28. En cas de surpression, la paroi peut se briser au niveau de l'amincissement.

Bien entendu, l'invention n'est pas limitée aux exemples qui viennent d'être donnés. En particulier, on peut combiner entre elles différentes caractéristiques des divers modes de réalisation.

Dans toute la description, y compris les revendications, l'expression « comportant un » doit être comprise comme étant synonyme de « comportant au moins un », sauf si le contraire est spécifié.

## Revendications

1. Procédé pour l'application, notamment sur des fibres kératiniques, d'un produit cosmétique (P), y compris de soin, comportant des particules (F) de forme allongée telles que des fibres, ce produit étant contenu dans un dispositif de conditionnement, le procédé comportant les étapes suivantes :
- placer le dispositif de conditionnement dans un four à micro-ondes,
- élever la température du produit en le soumettant à un rayonnement micro-ondes à l'intérieur du four,
- appliquer le produit au moyen d'un applicateur.

2. Procédé selon la revendication 1, **caractérisé en ce que** le dispositif de conditionnement comporte l'applicateur et un récipient contenant le produit, l'applicateur étant préalablement détaché du récipient lors de la soumission du produit au rayonnement micro-ondes.

3. Procédé selon l'une quelconque des revendications précédentes, **caractérisé par le fait que** l'applicateur est maintenu hors du four lors de l'élévation en température du produit.

4. Procédé selon l'une quelconque des revendications précédentes, **caractérisé par le fait que** l'applicateur est disposé dans le four et soumis à un rayonnement micro-ondes en vue d'élever sa température au niveau d'une surface d'application.

5. Procédé selon l'une quelconque des revendications précédentes, **caractérisé par le fait que** le produit présente des propriétés qui permettent une application à chaud, après chauffage, ou une application à température ambiante, sans chauffage.

6. Procédé selon l'une quelconque des revendications précédentes, **caractérisé par le fait que** le produit est chauffé dans le four à micro-ondes de façon à être porté à une température comprise entre 30 °C et 80°C.

7. Procédé selon l'une quelconque des revendications précédentes, **caractérisé par le fait que** la durée pendant laquelle le produit est exposé au rayonnement micro-ondes est comprise entre 1 et 60 secondes, mieux entre 2 et 50 secondes, voire entre 3 et 25 secondes.

8. Procédé selon l'une quelconque des revendications précédentes, **caractérisé par le fait que** le dispositif présenté dans une position de chauffage est dans une position couchée, pour laquelle une hauteur de produit relativement à un plan sur lequel le dispositif est présenté est inférieure à une hauteur de produit lorsque le dispositif est présenté relativement à ce plan dans une position d'utilisation.

9. Dispositif de conditionnement apte à être chauffé dans un four à micro-ondes, **caractérisé par le fait qu'**il comporte un applicateur et un récipient contenant un produit cosmétique (P) comportant des particules (F) de forme allongée telles que des fibres.

10. Dispositif selon la revendication précédente, **caractérisé en ce que** les particules sont dispersées dans une composition de revêtement des fibres kératiniques.

11. Dispositif selon l'une des revendications 9 à 10 **caractérisé par le fait qu'**il comporte au moins un indicateur (40 ; 45 ; 46 ; 47 ; 170) sensible à la température du produit.

12. Dispositif selon la revendication précédente, **caractérisé par le fait que** l'indicateur (40 ; 47) est solidaire du récipient contenant le produit.

13. Dispositif selon l'une des revendications 11 à 12 **caractérisé par le fait que** l'indicateur (40 ; 170) est solidaire de l'applicateur au moins lorsque le dispositif est placé dans le four à micro-ondes.

14. Dispositif selon l'une des revendications 11 à 13 **caractérisé par le fait que** l'indicateur (40 ; 170) est solidaire d'un organe de fermeture du récipient au moins lorsque le dispositif est placé dans le four à micro-ondes.

15. Dispositif selon l'une quelconque des revendications 11 à 14, **caractérisé par le fait que** l'indicateur (40 ; 45 ; 46 ; 47 ; 170) change de couleur avec la température, notamment autour d'une température de virage.

16. Dispositif selon l'une quelconque des revendications 11 à 15, **caractérisé par le fait que** l'indicateur change d'aspect de façon réversible avec la température.

17. Dispositif selon l'une quelconque des revendications 11 à 16, **caractérisé par le fait que** l'indicateur change d'état à une température inférieure à celle à laquelle on souhaite réchauffer le produit.

18. Dispositif selon l'une quelconque des revendications 11 à 17, **caractérisé par le fait que** l'indicateur (40) comporte au moins un support flexible fixé, notamment par collage, sur le dispositif.

19. Dispositif selon l'une quelconque des revendications 11 à 18, **caractérisé par le fait que** l'indicateur (46) est réalisé par impression sur le dispositif d'une encre comportant un pigment thermochromique.

20. Dispositif selon l'une quelconque des revendications 11 à 19, **caractérisé par le fait que** l'indicateur est formé par incorporation d'un pigment thermochromique dans la matière d'une partie (45 ; 47) au moins du dispositif.

21. Dispositif selon l'une quelconque des revendications 11 à 20, **caractérisé par le fait que** le récipient présente une épaisseur variable, et en ce que l'indicateur couvre au moins deux zones d'épaisseur distincte de ce récipient.

22. Dispositif selon l'une quelconque des revendications 9 à 21, **caractérisé par le fait qu'**il comporte deux indicateurs, chaque indicateur ayant une température de virage distincte.

23. Dispositif selon l'une des revendications 9 à 22, **caractérisé par le fait qu'**il comporte un organe (42 ; 165) de fermeture du récipient

24. Dispositif selon l'une des revendications 9 à 23, **caractérisé par le fait que** le réservoir est perméable aux micro-ondes.

25. Dispositif selon l'une des revendications 9 à 24, **caractérisé par le fait qu'**il comporte un repère (120) indiquant à l'utilisateur la position dans laquelle il doit être préférentiellement placé dans le four à micro-ondes.

26. Dispositif selon l'une des revendications 9 à 25, **caractérisé par le fait qu'**il comporte une paroi agencée pour permettre de le poser de manière stable en position couchée, notamment une paroi ayant une section transversale prismatique offrant au moins une surface plane.

27. Dispositif selon l'une des revendications 9 à 26, **caractérisé par le fait que** le dispositif comporte un signe (121) renseignant l'utilisateur sur la possibilité de le placer dans un four à micro-ondes.

28. Dispositif selon l'une des revendications 9 à 27, **caractérisé par le fait qu'**il comporte un organe anti-projection permettant de réduire le risque de projection de produit à l'ouverture du dispositif sous l'effet d'une surpression créée par le chauffage du produit, notamment à cause de la dilatation de l'air.

29. Dispositif selon l'une des revendications 9 à 28, **caractérisé par le fait qu'**il comporte un organe réducteur d'écoulement servant notamment à réduire le risque de perte de produit en cas de renversement accidentel du récipient.

30. Dispositif selon l'une des revendications 9 à 29, **caractérisé par le fait que** le produit contenu dans le récipient est liquide à température ambiante.

31. Dispositif selon l'une quelconque des revendications 9 à 30, **caractérisé par le fait qu'**il est dépourvu de métal.

32. Dispositif l'une quelconque des revendications 9 à 31, **caractérisé par le fait que** le récipient comporte une paroi (41) réalisée au moins en partie dans un premier matériau, et en ce qu'un isolant thermique (50 ; 60 ; 70 ; 80 ; 85) entoure au moins partiellement la surface extérieure du récipient.

33. Dispositif selon la revendication 32, **caractérisé par le fait que** l'isolant thermique (50 ; 60 ; 70 ; 80) est réalisé dans un deuxième matériau ayant une conductivité thermique inférieure à celle du premier matériau.

34. Dispositif selon l'une quelconque des revendications 9 à 33 **caractérisé par le fait qu'**il comporte une soupape de sécurité (150 ; 154 ; 176 ; 177) agencée pour s'ouvrir en cas de surpression dans le récipient.

35. Dispositif selon l'une quelconque des revendications 9 à 34 **caractérisé par le fait qu'**il comporte un rehausseur apte à placer le récipient plus haut relativement à un plan sur lequel il est posé.

36. Dispositif selon la revendication précédente, **caractérisé par le fait que** le rehausseur comporte un indicateur de température.
